# EUROPEAN PATENT APPLICATION

(11) **EP 4 624 450 A2**
(43) Date of publication of application: **01.10.2025**
(21) Application number: 25162514.1
(22) Date of filing: 28.01.2022
(51) Int. Cl.: C07C 235/60

(54) **NOVEL SYNTHESIS OF SALCAPROZIC ACID BY AMIDE FORMATION**

(30) Priority: 29.01.2021 EP 21154303
(62) Divisional of application: 22703590.4
(71) Applicant: Novo Nordisk A/S, 2880 Bagsvaerd (DK)
(72) Inventor: KROGSGAARD-LARSEN, Niels, 2880 Bagsværd (DK); RAUNKJÆR, Michael, 2880 Bagsværd (DK); RISINGER, Christian, Walter, 2880 Bagsværd (DK); TAANING, Rolf, Hejle, 2880 Bagsværd (DK); KOMNATNYY, Vitaly, 2880 Bagsværd (DK)

(57) **Abstract**

The present invention relates to a chemical process for the synthesis of the compound 8-[(2-hydroxybenzoyl)amino]octanoic acid or a derivative thereof useful in the process of making sodium 8-[(2-hydroxybenzoyl)amino]octanoate (SNAC), a delivery agent used in solid pharmaceutical compositions. The invention provides an efficient synthesis using less hazardous chemicals and/or a reproducible process that is easier to handle on production scale.

## Description

### TECHNICAL FIELD OF THE INVENTION

The present invention relates to an improved chemical process for the manufacturing of the compound 8-[(2-hydroxybenzoyl)amino]octanoic acid, which is a compound useful in the delivery of biologically and chemically active agents.

### BACKGROUND

Sodium N-(8[2-hydroxybenzoyl]-amino)caprylate, sometimes referred to as sodium 8-[(2-hydroxybenzoyl)amino]octanoate (SNAC) is a compound used as a delivery agent of chemical and biological active agents, such as protein- and peptide-based pharmaceuticals stimulating oral bioavailability of the biological active ingredient.

WO08028859 provides a method for producing SNAC from 8-[(2-hydroxybenzoyl)amino]octanoic acid.

The compound 8-[(2-hydroxybenzoyl)amino]octanoic acid is therefore a crucial intermediate in the manufacture of SNAC.

General preparations of 8-[(2-hydroxybenzoyl)amino]octanoic acid, are set out in WO00/46182 and WO00/59863, and further processes of manufacturing are provided in US5962710 and CN1446795.

However, there is still room for improving the processes for the preparation of 8-[(2-hydroxybenzoyl)amino]octanoic acid which should ideally be economically efficient, safe, sustainable and better suited for large scale production.

### SUMMARY

The present invention provides a manufacturing process of 8-[(2-hydroxybenzoyl)amino]octanoic acid or a derivative hereof. The method is environmentally friendly in relation to starting materials, solvents and/or by-products of the reaction. Also, or alternatively, the invention provides a cost-efficient and sustainable means of generating the 8-[(2-hydroxybenzoyl)amino]octanoic acid or a derivative hereof well suited for production scale.

This is achieved by starting from a compound of formula (II), preferably of formula (II-a), and reacting it with a compound of formula (III), preferably of formula (III-a), to obtain the compound of formula (I), preferably of formula (I-a), as depicted below and defined herein.

The overall reaction is depicted in the following SCHEMES 1 and 2:

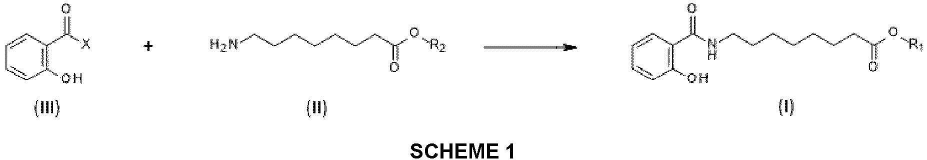

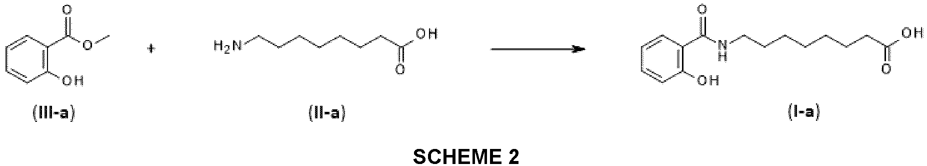

In a first aspect of the invention, a method for producing 8-[(2-hydroxybenzoyl)amino]octanoic acid or a derivative thereof comprising the step of reacting 8-amino caprylic acid or a derivative thereof with a salicylic acid derivative is provided.

In a second aspect of the invention, a method for producing a compound of formula (I), 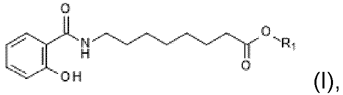
wherein R₁ is selected from hydrogen, C₁-C₆-alkyl, phenyl, benzyl, Li⁺, Na⁺ and K⁺, comprising the step of reacting the compound of formula (II), 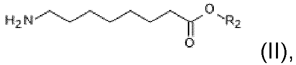
wherein R₂ is selected from hydrogen, C₁-C₆-alkyl, phenyl, benzyl, Li⁺, Na⁺ and K⁺, and the compound of formula (III), 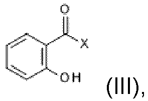
wherein X is selected from O-alkyl, O-phenyl, O-benzyl and NHS is provided.

In some embodiments, R₁ is chosen from hydrogen, C₁-C₆-alkyl, phenyl, benzyl, Li⁺, Na⁺ and K⁺.

In some embodiments, R₂ is chosen from hydrogen, C₁-C₆-alkyl, phenyl, benzyl, Li⁺, Na⁺ and K⁺.

In some embodiments, X is selected from O-alkyl, O-phenyl, O-benzyl and NHS.

Also, or alternatively, in a third aspect of the invention, a method for producing a compound of formula (I), preferably of formula (I-a), 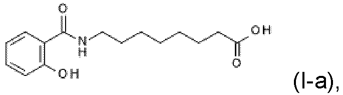
comprising the step of reacting the compound of formula (II), preferably of formula (II-a), 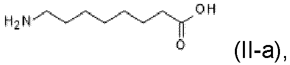
and the compound of formula (III), preferably of formula (III-a), 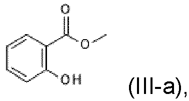 is provided.

An advantage of this process for the manufacture of the compound of formula (I), preferably of formula (I-a) is that it is economically efficient due to readily available starting materials. Another advantage is that it utilizes safe starting materials that make the invention well suited for a large production scale. Also, or alternatively, another advantage of the invention is that it provides sustainable means of manufacturing the compound of formula (I), in particular formula (I-a), without the generation of hazardous waste.

### BRIEF DESCRIPTION OF DRAWINGS

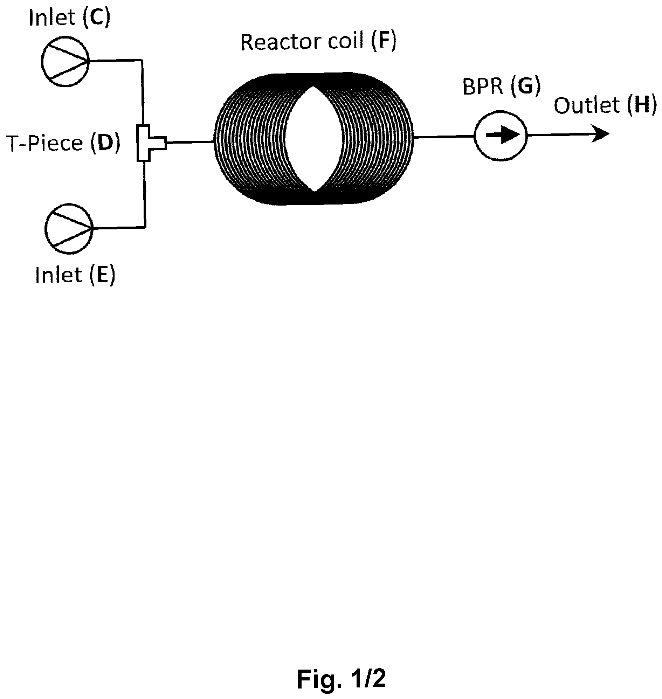

Figure 1 shows a plug flow reactor setup used in continuous flow synthesis. The system comprises of inlets (C) and (E), a connection point (T-piece) or mixer (D), reactor coil (F), back pressure regulator (G) and outlet (H). The units can be connected by use of tubing or other channel forming materials allowing for laminar or turbulent flow. The two inlets (C) and (E) can be used to inject the reactants or solutions into (D) to ensure appropriate mixing of components.

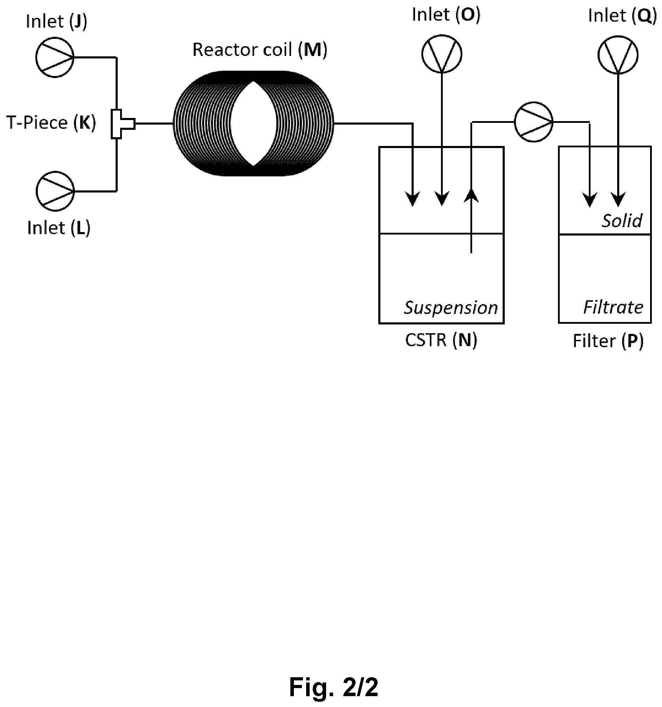

Figure 2 shows a setup used for precipitation and isolation in continuous flow synthesis. The reaction mixture (J or outlet from H) is mixed in (K) with aqueous base from (L) and passed through the reactor coil (M) into a CSTR (N) equipped with a stirrer. Solutions (aq. acid) can be continuously added via inlet (O). The resulting product mixture is continuously removed from the CSTR and transferred to a filter or centrifuge (P) for isolation. The solid product is washed (Q) before drying.

### General Terms

In what follows, Greek letters may be represented by their symbol or the corresponding written name, for example: α = alpha; β = beta; ε = epsilon; γ = gamma; ω = omega; etc. Also, the Greek letter of µ may be represented by "u", e.g. in µL = uL, or in µM = uM.

Unless otherwise indicated, terms presented in singular form generally also include the plural situation.

The term "compound" is used herein to refer to a molecular entity, and "compounds" may thus have different structural elements besides the minimum element defined for each compound or group of compounds.

Also described herein are compounds and methods in which open ended terms like "comprises" and "comprising" are replaced with closed terms such as "consists of", "consisting of", and the like.

Where the plural form is used for compounds, starting materials, intermediates, salts and the like, this intends to mean one (preferred) or more single compound(s), salt(s), intermediate(s) or the like, where the singular or the indefinite article ("a", "an") is used, this does not intend to exclude the plural, but only preferably means "one".

The compound name 8-[(2-hydroxybenzoyl)amino]octanoic acid maybe used interchangeable with the name *N*-(8[2-hydroxybenzoyl]-amino)caprylic acid

The compound name sodium *N*-(8[2-hydroxybenzoyl]-amino)caprylate (SNAC) may be used interchangeable with the name sodium 8-[(2-hydroxybenzoyl)amino]octanoate .

Alkyl is defined as a straight or branch (one or, if desired and possible, more times) carbon moiety, and is especially C₁-C₇-alkyl, preferably C₁-C₆-alkyl, more preferably C₁-C₄-alkyl. In an alternative the same is describe by -C₁₋₇, -C₁₋₆ and -C₁₋₄.

The terms "C₄-C₇-" and "C₁-C₆-" respectively, define a moiety with up to and including maximally 7 or 6 respectively, carbon atoms, said moiety being branched (one or more times) or straight-chained and bound via a terminal or non-terminal carbon. Nonlimiting examples of alkyls are methyl, ethyl, *n*-propyl, *iso-*propyl, *n*-butyl, *iso-*butyl, *sec*-butyl, *tert*-butyl*, n*-pentyl, 1-methylbutyl, 2-methylbutyl, *iso*-pentyl, 1-ethylpropyl, 1-methyl-*iso*-butyl, 1,1-dimethylpropyl, *tert*-pentyl, cyclopentyl and cyclohexyl.

The term phenyl refers to the group of aryls which is derived from an aromatic hydrocarbon, such as C₆-hydrocarbons and C₆-(R)-hydrocarbons, wherein R is a substituent such as, but not limited to, alkyl, phenyl, NO₂, NH₃⁺, CF₃, CCl₃, CHO, C(O)alkyl, COOH, C(O)NH₂, SO₃H, CN, F, Cl, Br, I, NHC(O)alkyl, NHC(O)phenyl, NH₂, OH.

The term benzyl refers to phenyl-CH₂-. The term carboxyl refers to -CO₂H. The term phenol refers to a benzene ring linked directly to a hydroxy (-OH) group.

The term metal is such as alkali metals, for example sodium, potassium, lithium, or magnesium or calcium.

Halo or halogen is preferably fluoro, chloro, bromo or iodo, most preferably chloro, bromo or iodo.

In view of the close relationship between the compounds in free form and in form of their salts, any reference to "compounds", "starting materials" and "intermediates" herein is also to be understood as referring to one or more salts of that compound, starting material or intermediate.

Salts can be formed where salt forming groups, such as basic or acidic groups, are present and can exist in dissociated form at least partially, e.g. in pH range from 2 to 12 in aqueous solutions or mixtures of water and organic solvents, or can be isolated especially in solid, especially crystalline, form. Such salts are formed, for example, with organic or inorganic acids, from compounds or any of the intermediates mentioned herein with a basic nitrogen atom (e.g. amino). Suitable inorganic salts are, for example, but not limited to, mineral acids, such as hydrochloric acid. Suitable organic salts are, for example, but not limited to, carboxylic salts containing groups such as acetic acid. In the presence of negatively charged groups, such as a carboxy group, salts may also be formed with bases, e.g. ammonium or metal salts, such as, but not limited to, sodium, magnesium, potassium or calcium salts. When a basic group and an acid group are present in the same molecule, any of the intermediates and compounds mentioned herein may also form internal salts such as zwitter ions.

The term "protecting group" comprises any group which is capable of reversibly protecting a functionality, such as a nitrogen or carboxyl functionality. Suitable protecting groups are conventionally used and are described e.g. in relevant chapters of standard reference books such as J. F. W. McOmie, "Protective Groups in Organic Chemistry", Plenum Press, London and New York, 1973. Nitrogen protecting groups comprise any groups which can reversibly protect a nitrogen functionality, preferably amine and/or amide protecting groups. Carboxyl protecting groups can be any groups known in the art, especially C₁-C₆-alkyl, such as ethyl or methyl or C₆-C₁₀-aryl-C₁-C₆-alkyl, such as benzyl.

### DESCRIPTION

The present invention provides a manufacturing process of the compound 8-[(2-hydroxybenzoyl)amino]octanoic acid using an environmentally friendly approach in relation to starting materials, solvents and/and by-products of the reaction, by using readily available chemicals and/or mild reaction conditions and generating less hazardous waste. Also, or alternatively, the present invention provides a cost-efficient and sustainable means of generating the compound of formula (I), in particular of formula (I-a), well suited for production scale.

This is achieved by starting from a compound of formula (II), preferably of formula (II-a), and reacting it with a compound of formula (III), or a salt, ester, solvate, complex or another derivative thereof, preferably of formula (III-a), to obtain the compound of formula (I), or a salt, ester, solvate, complex or another derivative thereof, preferably of formula (I-a), as depicted below and defined herein.

### Reactants and products

The reaction or method as set out above, may be divided in a synthetic procedure and a precipitation procedure. For the synthetic procedure, different reactants may be used to obtain the desired product, such as a compound of formula (I). The synthetic part of the method is also referred to as the reaction.

In a first aspect of the invention, a method for producing 8-[(2-hydroxybenzoyl)amino]octanoic acid or a derivative thereof comprising the step of reacting 8-amino caprylic acid or a derivative thereof with methyl salicylate or a derivative thereof is provided.

In a second aspect of the invention, a method for producing a compound of formula (I), 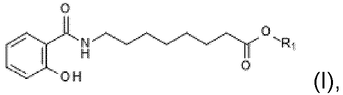
wherein R₁ is selected from hydrogen, C₁-C₆-alkyl, phenyl, benzyl, Mg²⁺, Ca²⁺, Li⁺, Na⁺ and K⁺,
comprising the step of reacting the compound of formula (II), 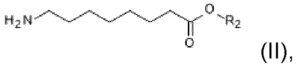
wherein R₂ is selected from hydrogen, C₁-C₆-alkyl, phenyl, benzyl, Mg²⁺, Ca²⁺, Li⁺, Na⁺ and K⁺,
and the compound of formula (III), 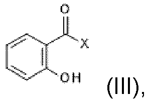
wherein X is selected from O-alkyl, O-phenyl, O-benzyl and NHS,
is provided.

In an embodiment of the invention, a compound of formula (I) is obtained from a compound of formula (II). In another embodiment of the invention, the compound of formula (II) is reacted with a compound of formula (III), to obtain the compound of formula (I).

In another embodiment of the invention, the compound of formula (II) is used to obtain the compound of formula (I).

In another embodiment of the invention, the compound of formula (III) is used to obtain the compound of formula (I).

In another embodiment of the invention, the compound of formula (II) is reacted with the compound of formula (III) to obtain the compound of formula (I).

Also, or alternatively, in a third aspect of the invention, a method for producing a compound of formula (I), 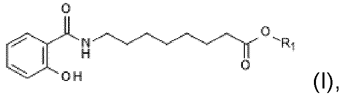
wherein R₁ is selected from hydrogen, C₁-C₆-alkyl, phenyl, benzyl, Mg²⁺, Ca²⁺, Li⁺, Na⁺ and K⁺, preferably of formula (I-a), 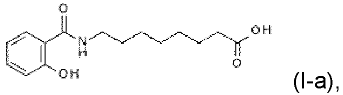
comprising the step of reacting the compound of formula (II), 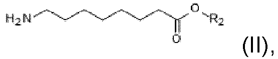
wherein R₂ is selected from hydrogen, C₁-C₆-alkyl, phenyl, benzyl, Mg²⁺, Ca²⁺, Li⁺, Na⁺ and K⁺,
preferably of formula (II-a), 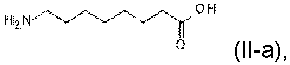
and the compound of formula (III), 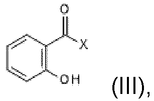
wherein X is selected from O-alkyl, O-phenyl, O-benzyl and NHS, preferably of formula (III-a), 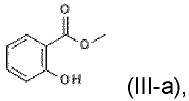 is provided.

In one embodiment of the invention, compound of formula (I), preferably of formula (I-a), is obtained from a compound of formula (II), preferably of formula (II-a). In another aspect of the invention, the compound of formula (II), preferably of formula (II-a), is reacted with a compound of formula (III), preferably of formula (III-a), to obtain the compound of formula (I), preferably of formula (I-a).

In another embodiment of the invention, the compound of formula (I-a), is obtained from a compound of formula (II-a). In another aspect of the invention, the compound of formula (II-a) is reacted with a compound of formula (III-a) to obtain the compound of formula (I), preferably of formula (I-a).

In another embodiment of the invention, the compound of formula (II), preferably of formula (II-a) is used to obtain the compound of formula (I), preferably of formula (I-a).

In another embodiment of the invention, the compound of formula (III), preferably of formula (III-a) is used to obtain the compound of formula (I), preferably of formula (I-a).

In another embodiment of the invention, the compound of formula (II-a) is reacted with the compound of formula (III-a), to obtain the compound of formula (I-a).

In some embodiments of the invention, R₁ may be selected from the group of hydrogen, C₁-C₆-alkyl, phenyl, benzyl, Li⁺, Na⁺ and K⁺. In some embodiments, R₂ may be selected from the group of hydrogen, C₁-C₆-alkyl, phenyl, benzyl, Li⁺, Na⁺ and K⁺. In some embodiments, R₁ and R₂ are, independently of each other, selected from the group of hydrogen, C₁-C₆-alkyl, phenyl, benzyl, Li⁺, Na⁺ and K⁺. In some embodiments of the invention, R₁ is selected from hydrogen, C₁-C₆-alkyl, phenyl and benzyl. In some embodiments of the invention, R₂ is selected from hydrogen, C₁-C₆-alkyl, phenyl and benzyl. In some embodiments of the invention, R₁ is hydrogen or K⁺. In some embodiments of the invention, R₂ is hydrogen or K⁺. In some embodiments of the invention, R₁ and R₂ are hydrogen or K⁺. In some embodiments, R₁ is hydrogen. In some embodiments, R₂ is hydrogen. In some embodiments, R₁ and R₂ are hydrogen. In some embodiments, R₁ is K⁺. In some embodiments, R₂ is K⁺.

In some embodiments, X is selected from the group of O-alkyl, O-phenyl, O-benzyl and NHS. In some embodiments, X is O-alkyl, such as O-C₁₋₆, O-C₁₋₅, O-C₁₋₄ or O-C₁₋₃, In some embodiments, X is O-alkyl, such as O-methyl or O-ethyl. In some embodiments, X is O-methyl.

Also, or alternatively, the compound of formula (III), may comprise modifications, such as a substituent on the aromatic group, which does not participate in the reaction and can be removed after the reaction, such that the result of the reaction is the same as without the modification.

Also, or alternatively, the compound of formula (III), may include a modification of the hydroxyl group of the phenol, wherein the modification, such as for example a protecting group, can be converted to the hydroxyl group after the reaction, such that the result of the reaction is the same as without the modification.

Also, or alternatively, in another embodiment, a method for producing a compound of formula (I), preferably of formula (I-a), comprising the step of reacting the compound of formula (II), preferably of formula (II-a), and the compound of formula (III), preferably of formula (III-a), is provided.

In some embodiments, the compound of formula (II) is 8-amino caprylic acid (II-a). In some embodiments, the compound of formula (III) is methyl salicylate (III-a).

In one embodiment, a method for producing a compound of formula (I-a), 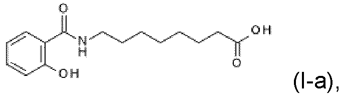
comprising the step of reacting the compound of formula (II-a), 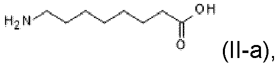
and the compound of formula (III-a), 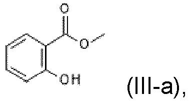
is provided.

### Base

In order to perform the synthetic part of the reaction or method, different bases known in the art can be used.

In one embodiment of the invention, the reaction occurs in the presence of a base. In one embodiment of the reaction, the compound of formula (II), preferably formula (II-a) and the compound of formula (III), preferably compound (III-a) is reacted with a base.

Also, or alternatively, in another embodiment, a method for producing a compound of formula (I-a), 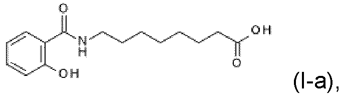
comprising the step of reacting the compound of formula (II-a), 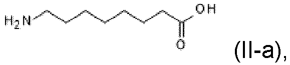
and the compound of formula (III-a), 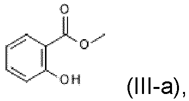
with a base, is provided.

In some embodiments of the invention, the base is selected from the group of NH₂-alkyl, NH-(alkyl)₂, *N*-(alkyl)₃, H₂N-alkyl-NH₂, piperidine, pyrrolidine, azepane, azocane, metal-OH, metal-O-alkyl, NaH, LiH, KH, Li-N(alkyl)₂, Li-alkyl. In another embodiment, the base is a metal-O-alkyl. In another embodiment, the base is potassium methoxide, KOMe.

### Solvents

The reaction as described above may be performed in a solution using a solvent to dissolve the reactants, i.e., the compounds of formula (II) and formula (III). Different solvents known in the art can be used depending on the specific reaction conditions.

In one embodiment of the invention, a method for producing 8-[(2-hydroxybenzoyl)amino]octanoic acid or a derivative thereof comprising the step of reacting 8-amino caprylic acid or a derivative thereof with methyl salicylate or a derivative thereof is provided, wherein the reaction occurs in solution.

Also, or alternatively, in another embodiment of the invention, the method comprises mixing a solution of the compound of formula (II), preferably of formula (II-a) with a solution of the compound of formula (III), preferably formula (III-a).

In some embodiments, the solvent is protic or aprotic, or a mixture thereof.

In some embodiments, the solvent is selected from the group of alcohols, nitromethane, acetonitrile, DMSO, sulfolane, DMF, NMP, ketones, halogenated solvents, ester-based solvents, ethers and hydrocarbons, and mixtures thereof. In some embodiments, the solvent is selected from the group of alcohols, such as methanol, ethanol, 1-propanol, 2-propanol, and DMSO or DMF, and mixtures thereof. In some embodiments, the solvent is methanol.

### Precipitation and isolation procedure

As described above, the reaction or method may be divided in a synthetic procedure and a precipitation procedure. Subsequent the synthetic procedure, different approaches may be used to precipitate and isolate the desired compound of formula (I), preferably of formula (I-a).

In one embodiment, a method for producing a compound of formula (I-a), 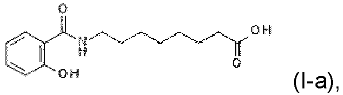 comprising the steps of
a) reacting the compound of formula (II-a), 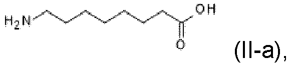 and the compound of formula (III-a), 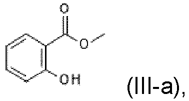 obtaining the compound of formula (I-b), 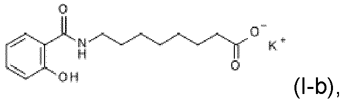 as an intermediate,
b) obtaining the compound of formula (I-a), is provided.

In one embodiment 8-[(2-hydroxybenzoyl)amino]octanoic acid, a derivative hereof, or the compound of formula (I), is obtained by precipitation.

In some embodiments, the purification of the compound of formula (I), 8-[(2-hydroxybenzoyl)amino]octanoic acid or a derivative thereof is performed in batch mode or by continuous precipitation.

In one embodiment of the methods described herein, an intermediate, such as a salt of 8-[(2-hydroxybenzoyl)amino]octanoic acid, is formed.

In one embodiment, the compound of formula (I-b), 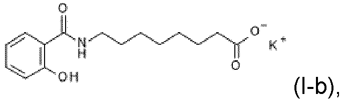 is formed as an intermediate in the reaction.

In one embodiment, the reaction or method may comprise a precipitation step to obtain the compound of formula (I), preferably of formula (I-a). In one embodiment, the compound of formula (I), preferably of formula (I-a), is formed by precipitation from water or from a mixture of water and an organic solvent. In one embodiment, the compound of formula (I), preferably of formula (I-a), is formed by precipitation from water or from a mixture of water and an organic solvent at pH < 7. In one embodiment, the pH is < 6. In one embodiment, the pH is < 5. In one embodiment, the pH is < 4.

In one embodiment the precipitation comprises the steps of
a) adding an aqueous base and/or
b) lowering the pH with an aqueous acid.

In one embodiment the aqueous base is added to the solution comprising the intermediate salt. In one embodiment, the aqueous base is potassium hydroxide, KOH.

In some embodiments, the precipitation and purification can be performed either in batch mode or by continuous precipitation.

In some embodiments, the compound of formula (I-a), 8-[(2-hydroxybenzoyl)amino]octanoic acid or a derivative thereof is collected in a continuous mode at the tube outlet. In some embodiments, 8-[(2-hydroxybenzoyl)amino]octanoic acid or a derivative thereof is isolated by filtration.

### Equipment

The reaction as set out above may be performed using various types of processes and equipment know in the art.

In some embodiments, the reaction is performed in batch mode. In some embodiments, the reaction is performed as a continuous process.

In some embodiments, the method is performed in batch mode. In some embodiments, the method is performed as a continuous process.

The term "batch mode" as used herein means a process wherein intermediates and products are handled in discrete unit operations affording intermediates and products in discrete amounts.

The term "continuous process" as used herein means a series of discrete unit operations wherein the output of one unit operation is the input of the next unit operation without interruption.

In some embodiments, the method is performed as a continuous process in a Plug Flow Reactor (PFR), such as a reactor coil. In some embodiments, the reaction is performed in a reactor coil. In one embodiment, a Teflon tubing is used as reactor coil. In some embodiments, at least one continuous stirred-tank reactor (CSTRs) is placed before or after the reactor coil.

In some embodiments, the method is performed as a continuous process comprising at least the steps of mixing in a reactor coil a solution 8-amino caprylic acid with a solution of methyl salicylate.

In some embodiments, the method is performed as a continuous process comprising at least a steps of mixing in a reactor coil a solution of the compound of formula (II), preferably of formula (II-a) and KOMe with a solution of the compound of formula (III), preferably of formula (III-a).

In some embodiments, the method is performed as a continuous process comprising at least a step of mixing in a reactor coil a solution 8-amino caprylic acid and KOMe with a solution of methyl salicylate.

The appropriate conversion of reactant and formation of product in a continuous flow process is controlled by the concentration of reactants, ratio between reactant flow rates, the residence time, temperature and pressure.

### Reaction conditions

The reaction as set out above may be performed using various reaction conditions know in the art, which may be optimized for each specific reaction and varied depending on the specific reactants, base, solvent and equipment used by the skilled artisan. As described above, the reaction or method can be performed either in batch mode or as a continuous process. As described herein, the two reactants (a compound of formula II and a compound formula III) are in an embodiment combined in solution forming a reaction mixture. In some embodiments of the invention, the reaction takes place at a temperature of 20 °C to 160 °C.

In some embodiment the reaction mixture is heated to reflux. In some embodiments of the invention, the reaction takes place at a temperature of 50 °C to 100 °C. In some embodiments, the reaction takes place at a temperature of 50 °C to 90 °C. In some embodiments, the reaction takes place at a temperature of 60 °C to 90 °C. In some embodiments of the invention where the reaction or method is performed in batch-mode, the reaction takes place under an inert atmosphere, such as under an atmosphere of nitrogen (N₂).

In some embodiments where the reaction or method is performed in a flow system, the temperature of the reactor coil is in the range from 20 °C to 180 °C. In some embodiments, the temperature of the reactor coil is in the range from 100 °C to 180 °C. In some embodiments, the temperature of the reactor coil is in the range from 100 °C to 150 °C. In some embodiments, the temperature of the reactor coil is in the range from 120 °C to 150 °C. In some embodiments, the temperature of the reactor coil is in the range from 110 °C to 130 °C. In some embodiments, the temperature of the reactor coil is at least 100 °C, such as 120 °C.

In some embodiments, the pressure of the reactor coil is in the range from 5 bar to 50 bar. In some embodiments, the pressure of the reactor coil is in the range from 10 bar to 25 bar. In some embodiments, the pressure of the reactor coil is in the range from 10 bar to 20 bar. In some embodiments, the pressure of the reactor coil is in the range from 10 bar to 15 bar. In some embodiments, the pressure of the reactor coil is 12 bar.

The term "residence time" used herein is defined as reactor volume divided by flow rate.

In some embodiments, the residence time in the reactor coil is in the range from 2 minutes to 120 minutes. In some embodiments, the residence time in the reactor coil is in the range from 20 minutes to 90 minutes. In some embodiments, the residence time in the reactor coil is in the range from 20 minutes to 60 minutes. In some embodiments, the residence time in the reactor coil is in the range from 20 minutes to 40 minutes. In some embodiments, the residence time is 60 min. In some embodiments, the residence time in the reactor coil is 30 minutes. In some embodiments, the residence time is 15 min.

In some embodiments, 8-[(2-hydroxybenzoyl)amino]octanoic acid or a derivative thereof is collected in a continuous mode. In some embodiments, the purification of 8-[(2-hydroxybenzoyl)amino]octanoic acid or a derivative thereof is performed in batch mode or by continuous precipitation.

In some embodiments, the product is precipitated from water, organic solvent or from a mixture of water and an organic solvent. In some embodiments, the product is precipitated from water or from a mixture of water and an organic solvent at pH < 7. In some embodiments, the product is precipitated from water or from a mixture of water and an organic solvent at pH < 6. In some embodiments, the product is precipitated from water or from a mixture of water and an organic solvent at pH < 5. In some embodiments, the product is precipitated from water or from a mixture of water and an organic solvent at pH < 4.

### Sodium N-(8[2-hydroxybenzoyl]-amino)caprylate (SNAC) and compositions comprising SNAC

Sodium *N*-(8[2-hydroxybenzoyl]-amino)caprylate (SNAC) is an excipient used in pharmaceutical compositions to enhance the bioavailability of an active pharmaceutical ingredient, such as chemical and biological active agents, such as protein- and peptide-based pharmaceuticals. SNAC can be prepared from 8-[(2-hydroxybenzoyl)amino]octanoic acid by methods known in the art.

The invention in a further aspect relates to a method for producing sodium N-(8[2-hydroxybenzoyl]-amino)caprylate (SNAC), comprising a step of producing a compound of formula (I), such as 8-[(2-hydroxybenzoyl)amino]octanoic acid as described herein.

In an embodiment, the method comprises
a) producing a compound of formula (I), such as 8-[(2-hydroxybenzoyl)amino]octanoic acid as described herein and
b) producing sodium *N*-(8[2-hydroxybenzoyl]-amino)caprylate (SNAC), using the compound of formula (I), such as 8-[(2-hydroxybenzoyl)amino]octanoic acid, obtained in a).

In an embodiment, the method comprises the steps of;
a) producing a compound of formula (I), such as 8-[(2-hydroxybenzoyl)amino]octanoic acid as described herein and
b) mixing a compound of formula (I), such as 8-[(2-hydroxybenzoyl)amino]octanoic acid, obtained in a) with 2-propanol and aqueous sodium hydroxide and
c) precipitating sodium *N*-(8[2-hydroxybenzoyl]-amino)caprylate (SNAC) from the mixture of b).

The invention in a further aspect relates to a method of producing a solid pharmaceutical composition, comprising the steps of;
a) producing a compound of formula (I), such as 8-[(2-hydroxybenzoyl)amino]octanoic acid as described herein,
b) producing sodium *N*-(8[2-hydroxybenzoyl]-amino)caprylate (SNAC) using the compound of formula (I), such as 8-[(2-hydroxybenzoyl)amino]octanoic acid, obtained in a) and
c) preparing said solid pharmaceutical compositions using the sodium *N*-(8[2-hydroxybenzoyl]-amino)caprylate obtained in step b).

### PARTICULAR EMBODIMENTS

1. A method for producing 8-[(2-hydroxybenzoyl)amino]octanoic acid or a derivative thereof comprising the step of reacting 8-amino caprylic acid or a derivative thereof with a salicylic acid derivative.
2. A method for producing a compound of formula (I), 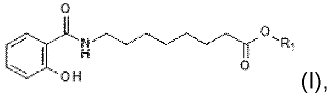
   wherein R₁ is selected from hydrogen, C₁-C₆-alkyl, phenyl, benzyl, Mg²⁺, Ca²⁺, Li⁺, Na⁺ and K⁺,
   comprising a step of reacting a compound of formula (II), 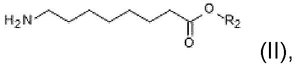
   wherein R₂ is selected from hydrogen, C₁-C₆-alkyl, phenyl, benzyl, Mg²⁺, Ca²⁺, Li⁺, Na⁺ and K⁺,
   and a compound of formula (III), 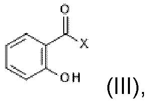
   wherein X is selected from O-alkyl, O-phenyl, O-benzyl and NHS.
3. The method according to embodiment 2, wherein R₁ is selected from hydrogen, C₁-C₆-alkyl, phenyl and benzyl.
4. The method according to embodiment 2 or 3, wherein R₂ is selected from hydrogen, C₁-C₆-alkyl, phenyl and benzyl.
5. The method according to any of the embodiments 2-4, wherein R₁ is hydrogen or K⁺.
6. The method according to any of the embodiments 2-5, wherein R₂ is hydrogen or K⁺.
7. The method according to any of the embodiments 2-6, wherein R₁ is hydrogen.
8. The method according to any of the embodiments 2-7, wherein R₂ is hydrogen.
9. The method according to embodiment 2-4, wherein R₁ and R₂ are hydrogen.
10. The method according to any of the embodiments 2-6 and 8, wherein R₁ is K⁺.
11. The method according to any of the embodiment 2-7 and 9-10, wherein R₂ is K⁺.
12. The method according to any of the embodiment 2-11, wherein X is O-alkyl, such as O-methyl or O-ethyl.
13. The method according to embodiment 12, wherein X is O-methyl.
14. The method according to embodiment 2, wherein the compound of formula (II) is 8-amino caprylic acid (formula II-a), 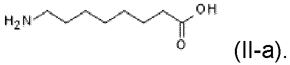
15. The method according to embodiment 2 or 14, wherein the compound of formula (III) is methyl salicylate (formula III-a), 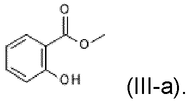
16. The method according to embodiment 2, 14 or 15, wherein the compound of formula (I) is 8-[(2-hydroxybenzoyl)amino]octanoic acid of formula (I-a), 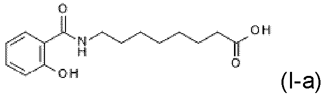
17. The method according to embodiment 2, 14 or 15, wherein the compound of formula (I) is potassium 8-[(2-hydroxybenzoyl)amino]octanoate of formula (I-b), 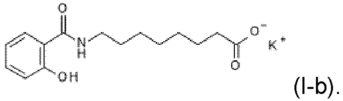
18. A method for producing a compound of formula (I), 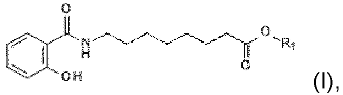
   wherein R₁ is selected from hydrogen, C₁-C₆-alkyl, phenyl, benzyl, Mg²⁺, Ca²⁺, Li⁺, Na⁺ and K⁺, preferably of formula (I-a), 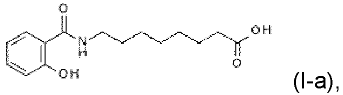
   comprising the step of reacting the compound of formula (II), 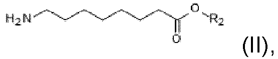
   wherein R₂ is selected from hydrogen, C₁-C₆-alkyl, phenyl, benzyl, Mg²⁺, Ca²⁺, Li⁺, Na⁺ and K⁺, preferably of formula (II-a), 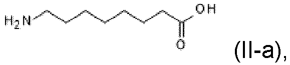
   and the compound of formula (III), 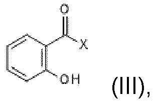
   wherein X is selected from O-alkyl, O-phenyl, O-benzyl and NHS, preferably of formula (III-a), 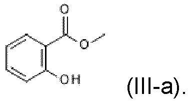
19. A method for producing a compound of formula (I-a), 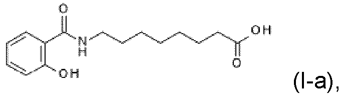
   comprising the step of reacting a compound of formula (II-a), 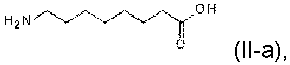
   and the compound of formula (III-a), 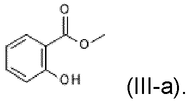
20. The method according to any of the previous embodiments, wherein the reaction occurs in the presence of a base.
21. The method according to embodiment 20, wherein the base is selected from the group of NH₂-alkyl, NH-(alkyl)₂, N-(alkyl)₃, H₂N-alkyl-NH₂, piperidine, pyrrolidine, azepane, azocane, metal-OH, metal-O-alkyl, NaH, LiH, KH, Li-N(alkyl)₂, Li-alkyl.
22. The method according to embodiment 20, wherein the base is a metal-O-alkyl.
23. The method according to embodiment 20, wherein the base is potassium methoxide, (KOMe).
24. The method according to embodiment 1, for producing 8-[(2-hydroxybenzoyl)amino]octanoic acid comprising the step of reacting 8-amino caprylic acid with methyl salicylate.
25. The method according to any of the previous embodiments, wherein the reaction occurs in solution.
26. The method according to any of the previous embodiments, wherein the method comprises mixing a solution of the compound of formula (II), preferably of formula (II-a) with a solution of the compound of formula (III), preferably formula (III-a).
27. The method according to any of the previous embodiments, wherein a solution of the compound of formula (II), preferably of formula (II-a) is used.
28. The method according to any of the previous embodiments, wherein a solution of the compound of formula (III), preferably of formula (III-a) is used.
29. The method according to any of the previous embodiments, wherein the reactants (the compound of formula (II) and the compound of formula (III)), are dissolved in one or more solvents.
30. The method according to embodiments 26-29, wherein the solvent(s) is protic or aprotic, or a mixture thereof.
31. The method according to embodiments 26-29, wherein the solvent(s) is selected from the group of alcohols, nitromethane, acetonitrile, DMSO, sulfolane, DMF, NMP, ketones, halogenated solvents, ester-based solvents, ethers and hydrocarbons, and mixtures thereof.
32. The method according to embodiment 26-29, wherein the solvent(s) is selected from the group of alcohols, such as methanol, ethanol, 1-propanol, 2-propanol, and DMSO or DMF, and mixtures thereof.
33. The method according to embodiment 26-29, wherein the solvent(s) is methanol.
34. The method according to any of the previous embodiments, wherein the precipitation or purification of 8-[(2-hydroxybenzoyl)amino]octanoic acid or a derivative thereof is performed in batch mode or by continuous precipitation.
35. The method according to any of the previous embodiments, for producing a compound of formula (I-a), wherein a salt of 8-[(2-hydroxybenzoyl)amino]octanoic acid, such the compound of formula (I-b), is formed as an intermediate.
36. The method according to any of the embodiment 1-35, wherein the compound of formula (I), preferably of formula (I-a), is obtained by precipitation.
37. A method for producing a compound of formula (I-a), 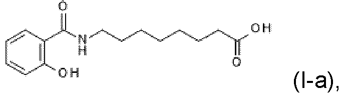 comprising the steps of
   a) reacting the compound of formula (II-a), 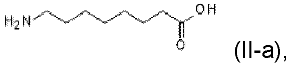 and the compound of formula (III-a), 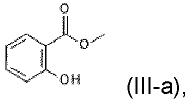 obtaining the compound of formula (I-b), 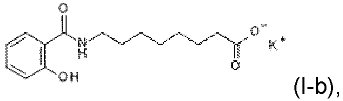 as an intermediate, and
   b) obtaining the compound of formula (I-a) by precipitation.
38. The method according to embodiments 36 or 37, wherein the compound of formula (I), preferably of formula (I-a), is formed by precipitation from water or from a mixture of water and an organic solvent.
39. The method according to embodiments 36 or 37, wherein the compound of formula (I), preferably of formula (I-a), is formed by precipitation from water or from a mixture of water and an organic solvent at pH < 7.
40. The method according to any of the embodiments 36-39, wherein the precipitation comprises the steps of
   a) adding an aqueous base and/or
   b) lowering the pH with an aqueous acid.
41. The method according to embodiment 40, wherein the aqueous. base is potassium hydroxide, KOH.
42. The method according to any of the previous embodiments, wherein the compound of formula (I), 8-[(2-hydroxybenzoyl)amino]octanoic acid or a derivative thereof is collected in a continuous mode at the tube outlet.
43. The method according to any of the previous embodiments, wherein the compound of formula (I), 8-[(2-hydroxybenzoyl)amino]octanoic acid or a derivative thereof is isolated by filtration.
44. The method according to any of the previous embodiments, wherein the method is performed in batch mode.
45. The method according to embodiment 44, wherein the reaction takes place at a temperature of 20 °C to 160 °C.
46. The method according to embodiment 44, wherein the reaction takes place at a temperature of 50 °C to 100 °C.
47. The method according to embodiment 44, wherein the reaction takes place at a temperature of 50 °C to 90 °C.
48. The method according to embodiment 44, wherein the reaction takes place at a temperature of 60 °C to 90 °C.
49. The method according to embodiment 44, wherein the reaction mixture is heated to reflux.
50. The method according to any of the previous embodiments, wherein the reaction takes place under an atmosphere of nitrogen (N₂).
51. The method according to any of the previous embodiments, wherein the method is performed as a continuous process.
52. The method according to any of the previous embodiments, wherein the reaction takes place in a Plug Flow Reactor, such as a reactor coil.
53. The method according to embodiment 50, wherein Teflon tubing is used as reactor coil.
54. The method according to any one of embodiments 49-50, wherein at least one continuous stirred-tank reactor (CSTRs) is placed before or after the reactor coil.
55. The method according to any of the previous embodiments, wherein the method is performed as a continuous process comprising at least the steps of mixing in a reactor coil a solution of the compound of formula (II), preferably of formula (II-a) with a solution of the compound of formula (III), preferably of formula (III-a).
56. The method according to any of the previous embodiments, wherein the method is performed as a continuous process comprising at least the steps of mixing in a reactor coil a solution 8-amino caprylic acid with a solution of methyl salicylate.
57. The method according to any of the previous embodiments, wherein the method is performed as a continuous process comprising at least the steps of mixing in a reactor coil a solution of the compound of formula (II), preferably of formula (II-a) and KOMe with a solution of the compound of formula (III), preferably of formula (III-a).
58. The method according to any of the previous embodiments, wherein the method is performed as a continuous process comprising at least the steps of mixing in a reactor coil a solution 8-amino caprylic acid and KOMe with a solution of methyl salicylate.
59. The method according to any of the embodiments 49-56, wherein the temperature of the reactor coil is in the range from 20 °C to 180 °C.
60. The method according to any of the embodiments 49-56, wherein the temperature of the reactor coil is in the range from 100 °C to 180 °C.
61. The method according to any of the embodiments 49-56, wherein the temperature of the reactor coil is in the range from 100 °C to 150 °C.
62. The method according to any of the embodiments 49-56, wherein the temperature of the reactor coil is in the range from 120 °C to 150 °C.
63. The method according to any of the embodiments 49-56, wherein the temperature of the reactor coil is in the range from 110 °C to 130 °C.
64. The method according to any of the embodiments 49-56, wherein the temperature of the reactor coil is at least 100 °C, such as 120 °C.
65. The method according to any of the embodiments 49-62, wherein the pressure of the reactor coil is in the range from 5 bar to 50 bar.
66. The method according to any of the embodiments 49-62, wherein the pressure of the reactor coil is in the range from 10 bar to 25 bar.
67. The method according to any of the embodiments 49-62, wherein the pressure of the reactor coil is in the range from 10 bar to 20 bar.
68. The method according to any of the embodiments 49-62, wherein the pressure of the reactor coil is in the range from 10 bar to 15 bar.
69. The method according to any of the embodiments 49-62, wherein the pressure of the reactor coil is 12 bar.
70. The method according to any of the embodiments 49-67, wherein the residence time in the reactor coil is in the range from 2 minutes to 120 minutes.
71. The method according to any of the embodiments 49-67, wherein the residence time in the reactor coil is in the range from 20 minutes to 90 minutes.
72. The method according to any of the embodiments 49-67, wherein the residence time in the reactor coil is in the range from 20 minutes to 60 minutes.
73. The method according to any of the embodiments 49-67, wherein the residence time in the reactor coil is in the range from 20 minutes to 40 minutes.
74. The method according to any of the embodiments 49-67, wherein the residence time in the reactor coil is 60 minutes.
75. The method according to any of the embodiments 49-67, wherein the residence time in the reactor coil is 30 minutes.
76. The method according to any of the embodiments 49-67 wherein the residence time in the reactor coil is 15 minutes.
77. A method for producing sodium N-(8[2-hydroxybenzoyl]-amino)caprylate (SNAC), comprising a step of producing a compound of formula (I), such as 8-[(2-hydroxybenzoyl)amino]octanoic acid, according to any of the previous embodiments.
78. A method for producing sodium N-(8[2-hydroxybenzoyl]-amino)caprylate (SNAC), comprising the steps of
   a) producing a compound of formula (I), such as 8-[(2-hydroxybenzoyl)amino]octanoic acid, according to any of the embodiments 1-74 and
   b) producing sodium N-(8[2-hydroxybenzoyl]-amino)caprylate (SNAC), using the compound of formula (I), such as 8-[(2-hydroxybenzoyl)amino]octanoic acid, obtained in a).
79. A method for producing sodium N-(8[2-hydroxybenzoyl]-amino)caprylate (SNAC), comprising the steps of
   a) producing a compound of formula (I), such as 8-[(2-hydroxybenzoyl)amino]octanoic acid, according to any of the embodiments 1-74,
   b) mixing a compound of formula (I), such as 8-[(2-hydroxybenzoyl)amino]octanoic acid, obtained in a) with 2-propanol and aqueous sodium hydroxide and
   c) precipitating sodium N-(8[2-hydroxybenzoyl]-amino)caprylate (SNAC) from the mixture of b).
80. A method of producing a solid pharmaceutical composition, comprising the steps of
   a) producing a compound of formula (I), such as 8-[(2-hydroxybenzoyl)amino]octanoic acid, according to any of the embodiments 1-74,
   b) producing sodium N-(8[2-hydroxybenzoyl]-amino)caprylate (SNAC) using the compound of formula (I), such as 8-[(2-hydroxybenzoyl)amino]octanoic acid, obtained in a) and
   c) preparing said solid pharmaceutical compositions using the sodium N-(8[2-hydroxybenzoyl]-amino)caprylate obtained in step b).
81. A method of producing a solid pharmaceutical composition comprising sodium N-(8[2-hydroxybenzoyl]-amino)caprylate (SNAC), comprising the steps of
   a) producing sodium N-(8[2-hydroxybenzoyl]-amino)caprylate (SNAC) according to any of the embodiments 75-77,
   b) preparing said solid pharmaceutical compositions using the sodium N-(8[2-hydroxybenzoyl]-amino)caprylate (SNAC) obtained in step a).

### FURTHER EMBODIMENTS

1. A method for producing a compound of formula (I), 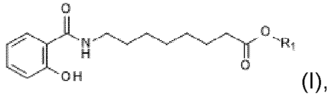
   wherein R₁ is selected from hydrogen, C₁-C₆-alkyl, phenyl, benzyl, Mg²⁺, Ca²⁺, Li⁺, Na⁺ and K⁺,
   comprising a step of reacting a compound of formula (II), 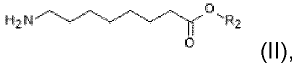
   wherein R₂ is selected from hydrogen, C₁-C₆-alkyl, phenyl, benzyl, Mg²⁺, Ca²⁺, Li⁺, Na⁺ and K⁺,
   and a compound of formula (III), 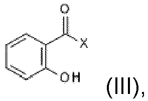
   wherein X is selected from O-alkyl, O-phenyl, O-benzyl and NHS.
2. The method according to embodiment 1, wherein R₁ is hydrogen or K⁺.
3. The method according to embodiment 1 or embodiment 2, wherein R₂ is hydrogen or K⁺.
4. The method according to any of the embodiments 1-3, wherein X is O-alkyl, such as O-methyl or O-ethyl.
5. The method according to any of the embodiments 1-3, wherein X is O-methyl.
6. The method according to embodiment 1 for producing a compound of formula (I-a), 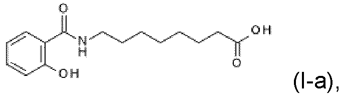
   comprising the step of reacting a compound of formula (II-a), 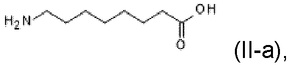
   and the compound of formula (III-a), 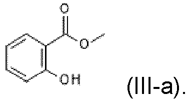
7. The method according to embodiment 1, wherein the compound of formula (I) is potassium 8-[(2-hydroxybenzoyl)amino]octanoate of formula (I-b), 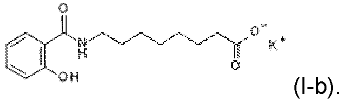
8. A method for producing a compound of formula (I-a), 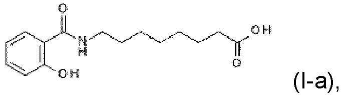 comprising the steps of
   a) reacting the compound of formula (II-a), 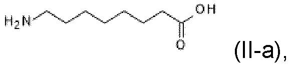 and the compound of formula (III-a), 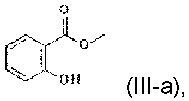 obtaining the compound of formula (I-b), 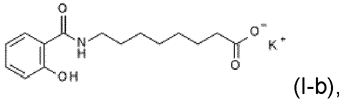 as an intermediate, and
   b) obtaining the compound of formula (I-a) by precipitation.
9. The method according to any of the previous embodiments, wherein the method is performed in batch mode.
10. The method according to any of the previous embodiments, wherein the method is performed as a continuous process.
11. A method for producing sodium *N*-(8[2-hydroxybenzoyl]-amino)caprylate (SNAC), comprising a step of producing a compound of formula (I), such as 8-[(2-hydroxybenzoyl)amino]octanoic acid, according to any of the previous embodiments.
12. A method for producing sodium *N*-(8[2-hydroxybenzoyl]-amino)caprylate (SNAC), comprising the steps of
   a) producing a compound of formula (I), such as 8-[(2-hydroxybenzoyl)amino]octanoic acid, according to any of the embodiments 1-9 and
   b) producing sodium *N*-(8[2-hydroxybenzoyl]-amino)caprylate (SNAC), using the compound of formula (I), such as 8-[(2-hydroxybenzoyl)amino]octanoic acid, obtained in a).
13. A method for producing sodium *N*-(8[2-hydroxybenzoyl]-amino)caprylate (SNAC), comprising the steps of
   a) producing a compound of formula (I), such as 8-[(2-hydroxybenzoyl)amino]octanoic acid, according to any of the embodiments 1-10,
   b) mixing a compound of formula (I), such as 8-[(2-hydroxybenzoyl)amino]octanoic acid, obtained in a) with 2-propanol and aqueous sodium hydroxide and
   c) precipitating sodium *N*-(8[2-hydroxybenzoyl]-amino)caprylate (SNAC) from the mixture of b).
14. A method of producing a solid pharmaceutical composition, comprising the steps of
   a) producing a compound of formula (I), such as 8-[(2-hydroxybenzoyl)amino]octanoic acid, according to any of the embodiments 1-10,
   b) producing sodium *N*-(8[2-hydroxybenzoyl]-amino)caprylate (SNAC) using the compound of formula (I), such as 8-[(2-hydroxybenzoyl)amino]octanoic acid, obtained in a) and
   c) preparing said solid pharmaceutical compositions using the sodium *N*-(8[2-hydroxybenzoyl]-amino)caprylate obtained in step b).
15. A method of producing a solid pharmaceutical composition comprising sodium *N*-(8[2-hydroxybenzoyl]-amino)caprylate (SNAC), comprising the steps of
   a) producing sodium *N*-(8[2-hydroxybenzoyl]-amino)caprylate (SNAC) according to any of the embodiments 11-13,
   b) preparing said solid pharmaceutical compositions using the sodium *N*-(8[2-hydroxybenzoyl]-amino)caprylate (SNAC) obtained in step a).

### EXAMPLES

The following examples serve to illustrate the invention without limiting the scope thereof, while they on the other hand represent preferred embodiments of the reaction steps, intermediates and/or the process of the present invention.

### List of Abbreviations

- aq.: Aqueous
- Ac: Acetyl
- 8-AOA: 8-Amino caprylic acid, 8-amino octanoic acid
- BPR: Back pressure regulator
- CSTR: Continuous stirred-tank reactor
- DMF: *N,N*-Dimethylformamide
- DMSO: Dimethyl sulfoxide
- Et: Ethyl
- eq.: Equivalent(s)
- h: Hour(s)
- HESI: Heated Electrospray Ionization
- HPLC: High Performance Liquid Chromatography
- HR: High Resolution
- *i*-Pr: *iso*-propyl
- LC-MS: Liquid Chromatography Mass Spectrometry
- Me: Methyl
- MeSA: Methyl salicylate
- min: Minute(s)
- NHS: *N*-hydroxysuccinimide ester
- NMP: *N*-Methyl-2-pyrrolidone
- NMR: Nuclear Magnetic Resonance
- *n*-Pr: Normal propyl
- PFR: Plug flow reactor
- Q-NMR: Quantitative Nuclear Magnetic Resonance
- SA: Salicylic acid

In quoting NMR data, the following abbreviations are used:
- s: singlet
- d: doublet
- t: triplet
- q: quartet
- quint.: quintet
- m: multiplet
Note that in the Examples, the numbers of compounds, such as Chem. 1, are different than the numbers representing the compounds in the description and in the embodiments, such as (I) or (II), just to clarify paradigmatically.

### General Methods of Preparation

The synthesis of 8-[(2-hydroxybenzoyl)amino]octanoic acid has been performed in two different ways, either via a batch procedure (parts A1 and A2 described in Method A) or via a continuous flow process (parts B1 and B2 described in Method B) or a combination of the procedures described in Method A and B

### Plug Flow Reactor setup

For the reaction performed as a continuous flow process, a plug flow reactor setup is used.

Figure 1 shows a plug flow reactor setup used in continuous flow synthesis (Part B1 of the method B described below).

Figure 2 shows a setup used for precipitation and isolation in continuous flow synthesis (Part B2 of the method described B below).

### General Method A for batch procedure:

### Part A1 - Synthesis:

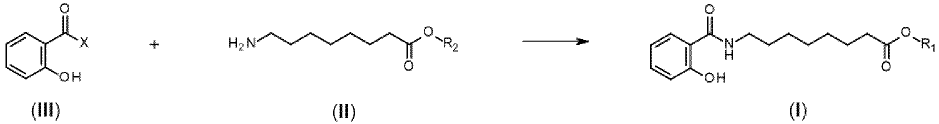

8-AOA derivative (Compound II, 1.00 eq.) was suspended in a solution of base (3.20 eq.) and organic solvent under an atmosphere of nitrogen (N₂). The mixture was heated to an appropriate temperature and added salicylic acid (SA) derivative (Compound III, 1.25 eq.) in one portion. The mixture was stirred at this temperature until a satisfying yield of compound (I) was obtained by IPC (HPLC)..

The product mixture was further processed by use of either Method A2 or Method B2 (see descriptions below).

### Part A2 - Precipitation and isolation:

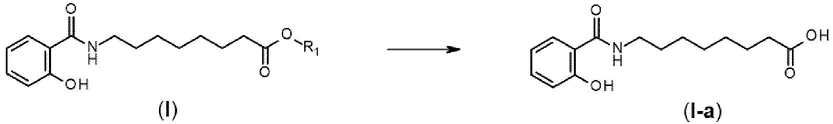

The temperature of the product mixture from either Method A1 or B1 was adjusted to an appropriate temperature and Milli-Q water was added to the mixture. In cases where a solvent swap was needed to facilitate precipitation the mixture was added water, concentrated *in vacuo,* and added the intended organic solvent followed by temperature adjustment prior to precipitation. The pH value was adjusted using aqueous acid precipitating compound (I-a). The resulting suspension was filtered and the isolated solid was washed successively with Milli-Q water. After crude drying on a filter the desired compound was dried at 40 °C until constant weight.

### General Method B for continuous flow process:

### Part B1 - Flow synthesis:

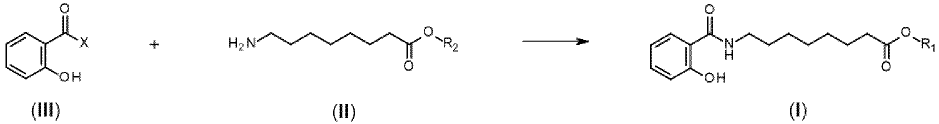

The synthesis of compound (I) using continuous processing was performed in a reactor coil (F). The equipment was assembled using Teflon tubing. The reactor coil (F) was emerged in an oil-bath.

8-AOA derivative (II) (1.00 eq.) dissolved in base (2.5 eq.) and solvent were mixed in a feed vessel and heated to an appropriate temperature. The mixture entered the system via inlet (C) and was mixed with salicylic acid (SA) derivative (III) (1.10 eq.) via inlet (E) in a T-piece (D) at appropriate temperature prior to passing through the reactor coil (F) at the reaction temperature with an appropriate residence time allowing for sufficient formation of product. A back-pressure regulator (G) was installed to maintain a constant pressure in the system.

Compound (I) was afterwards collected via outlet (H) for further processing by use of Method A2 or Method B2.

### Part B2 - Flow precipitation and isolation:

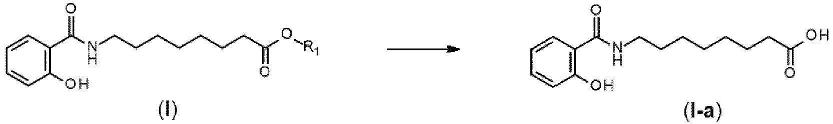

The synthesis of compound (I) using continuous processing was performed in a reactor coil (M). The equipment was assembled using Teflon tubing. The reactor coil (M) used for hydrolysis of excess salicylic acid (SA) derivative was emerged in an oil-bath.

The reaction mixture (1 eq.) obtained from either Method A1 or Method B1 entered the system via Inlet (J) and was mixed with aqueous base (2.70 eq.) entering the system via inlet (L) in a T-piece (K). The mixture was passed through the reactor coil (M) at an appropriate temperature and collected in a CSTR (N) allowing for a sufficient holding time. To the CSTR was continuously added aq. acid via inlet (O) maintaining the desired pH. The resulting suspension was transferred to a filter (P) and the crude product was isolated by filtration. The isolated solid was washed successively with Milli-Q water via Inlet (Q). After crude drying on the filter the product was dried at 40 °C until constant weight was achieved.

### Characterisation

### NMR spectroscopy

The NMR measurement was performed on a Bruker AV neo 400 MHz apparatus equipped with a BBO 5 mm cryoprobe. The spectrum was recorded at 298 K using 32 scans for protons, a delay time (D1) of 5 sec, and a 30-degree pulse to create observable magnetisation.

### LC-MS

The sample was dissolved in aqueous MeCN (50:50 MeCN:MilliQ water, 0.01 mg/mL) and thereafter subjected to HR LC-MS. The analysis was performed on a Q Exactive plus (ThermoFischer) equipped with a HESI source. The ion source was operated in the positive ion mode scanning from 95-700 Dalton with a resolution of 70000. Nitrogen was used as sheath and auxiliary gases. MS/MS was performed by HCD activation using an isolation width of 4.0 m/z and normalized stepped collision energy of 15, 30, 45.

### Example 1: Batch synthesis of potassium 8-[(2-hydroxybenzoyl)amino]octanoate (I-b) by use of Method A1:

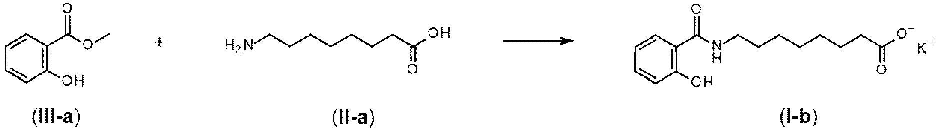

### Procedure 1:

8-AOA (II-a) (1000 g, 6.15 mol (assay corrected), 1.00 eq.) was weighed out in a 20L reactor and stored over night (18 h) under an atmosphere of N₂. The solid was suspended in 25 % KOMe in MeOH (4.00 L, 13.5 mol, 2.20 eq.) and transferred to a new reactor (Reactor 2). Reactor 1 was washed with 25 % KOMe in MeOH (1.82L, 6.16 mol, 1.00 eq.) and the resulting suspension transferred to Reactor 2. The mixture was heated to 40 °C and added MeSA (III-a) (1.00 L, 7.69 mol, 1.25 eq.) over the course of 2 min. The mixture was heated to reflux and stirred under an atmosphere of N₂ for 23 hours while the reaction was monitored by HPLC. The yield of compound (I-b) was found to be 95 % measured by HPLC (based on MeSA, SA, and compound (I-b)).

### Procedure 2:

8-AOA (II-a) (1000 g, 6.28 mol, 1.00 eq.) was suspended in a solution of 25 % KOMe in MeOH (3.00 L, 10.17 mol, 1.62 eq.) in a 30 L reactor under an atmosphere of N₂. Additional 25 % KOMe in MeOH (2.94 L, 9.92 mol, 1.58 eq.) were added. The mixture was heated to 40 °C and MeSA (III-a) (1.02 L, 7.69 mol, 1.25 eq.) was added over the course of 2 min. The mixture was heated to 60 °C and stirred under an atmosphere of N₂ for 23 hours while the reaction was monitored by HPLC. The yield of compound (I-b) was found to be 94 % measured by HPLC (based on MeSA, SA, and compound (I-b)).

### Example 2: Batch precipitation of 8-[(2-hydroxybenzoyl)amino]octanoic acid (I-a) by use of Method A2:

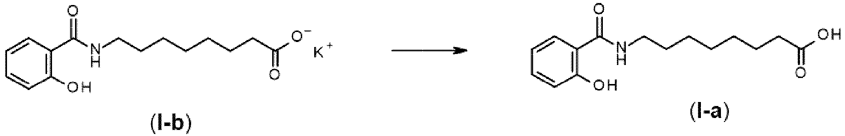

### Procedure 1:

The temperature of the product from method A1 was lowered to 50 °C and Milli-Q water (8.15 L) was added over the course of 5 min. The mixture is transferred the to a suitable container (20 L) and concentrated *in vacuo.* Pressure: 95-160 mBar, Water temperature: 50 °C) to a volume of 8.0 L. The mixture was transferred to a 20 L reactor and added i-PrOH (2.37 L, 30 % of the total volume). At room temperature (jacket temperature: 25 °C), the pH value was adjusted from pH 13.3 to 3.2 using conc. hydrochloric acid (1.75 L) over the course of 40 min. The mixture was stirred overnight (18 h) at 25 °C under an atmosphere of N₂. The precipitate was filtered through a Büchner funnel (d: 24 cm, h: 12 cm) in < 1 min. The reactor was washed with Milli-Q water (900 mL), which was also passed over the filter. The precipitate wash suspended in Milli-Q water and filtered 3 times (3 x 5.0 L water) followed by a crude drying on the filter, while continuing suction for 2 h. The precipitate was transferred to a tray and dried for 18 h at 40 °C and for 72 h at 21 °C to yield compound (I-a) (1643 g, 95 %) as slightly off-white solid.

In a combined workflow of using Method A1 (Example 1, procedure 1) + Method A2 (Example 2, procedure 1),a total yield of 95% of compound (I-a) was obtained.

### Procedure 2:

The temperature of the product from method A1 was lowered to 50 °C and Milli-Q water (1.95 L) was added over the course of 10 min. The mixture was stirred for 5 hours at 50 °C until HPLC shows a satisfying conversion of the excess MeSA to salicylic acid. At room temperature , the crude reaction mixture was diluted by the addition of mixture of water (1.70 L) and MeOH (3.25 L) before the pH value was adjusted from pH 13.5 to 3.2 using conc. HCl (1.80 L) over the course of 90 min. The mixture was stirred overnight (18 h) after an extra addition of water (0.12 L) at 25 °C under an atmosphere of N₂. The precipitate was filtered through a Büchner funnel. The filter cake was re-slurried, transferred to the reactor and triturated with a water/MeOH = 1:1 (w/w%) - mixture (8.50 L). The suspension was filtered through a Büchner funnel. The filter cake was re-slurried, transferred to the reactor and triturated with water (10 L) before a final filtration followed by a crude drying on the filter. The precipitate was transferred to a tray and dried for 48 h at 40 °C to yield compound (I-a) (1535 g, 88 %) as slightly off-white solid.

In a combined workflow of using Method A1 (Example 1, procedure 2) + Method A2 (Example 2, procedure 2), a total yield of 88% of compound (I-a) was obtained.

### Characterisation of compound 1-a obtained by Example 2

Exact mass by LC-MS [M+H]⁺ = 280,1541 Da, corresponding to C₁₅H₂₂O₄N⁺.

DMSO-d6; δ 1.30 (6H, m), 1.54 (4H, m), 2.20 (2H, t, ³J_{HH} = 7.4 Hz), 3.30 (2H, q, ³J_{HH} = 6.6 Hz), 6.90 (2H, m), 7.39 (H, td, ³J_{HH} = 7.7, 1.5 Hz), 7.85 (H, dd, ³J_{HH} = 7.4, 1.4 Hz), 8.80 (H, t, ³J_{HH} = 5.5 Hz), 12.00 (H, bs), 12.74 (H, s).

### Example 3: Continuous Flow synthesis of potassium 8-[(2-hydroxybenzoyl)amino]octanoate (I-b) by use of Method B1:

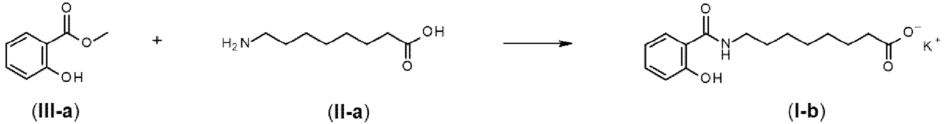

### Procedure 1:

8-AOA (II-a) (14.0 g, 87.9 mmol, 1.00 eq.) was dissolved in 25 % KOMe in MeOH (63.7 mL, 220 mmol, 2.50 eq.) and added MeOH (18.8 mL). The mixture was heated to 50 °C prior to reaction. The mixture of 8-AOA and base (477 µL/min, 0.40 mmol/min, 1.00 eq./min, 50 °C) was mixed with MeSA (III-a) (56.8 µL/min, 0.44 mmol/min, 1.10 eq./min, 21 °C) in a T-piece at 50 °C and pumped through the reactor coil at 120 °C and 30 min. residence time. A back pressure of 12 bar was applied.

The yield of product was found to be 85 % at steady state measured by HPLC (based on MeSA, SA, and product).

### Procedure 2:

8-AOA (II-a) (201.3 g, 1.26 mol, 1.00 eq.) was dissolved in 32 % KOMe in MeOH (697 mL, 3.16 mol, 2.50 eq.) and added MeOH (374 mL). The mixture was kept at ambient temperature prior to reaction. The mixture of 8-AOA and base (9.01 mL/min, 9.01 mmol/min, 1.00 eq./min) was mixed with MeSA (III-a) (1.40 mL/min, 10.8 mmol/min, 1.20 eq./min) in a T-piece at 180 °C and pumped through the reactor coil at 180 °C and 5 min. residence time. A back pressure of 26-27 bar was applied.

The yield of product was found to be 99 % at steady state measured by HPLC (based on 8-AOA).

### Example 4: Continuous precipitation of 8-[(2-hydroxybenzoyl)amino]octanoic acid (I-a) by use of Method B2:

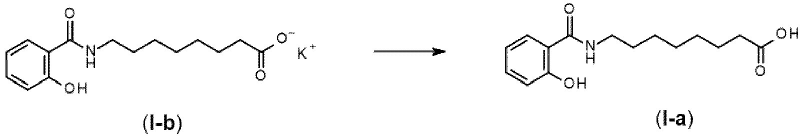

A reaction mixture (0.60 mM, 1.00 eq., 2.01 mL/min) obtained from Method A1 was mixed continuously with aq. KOH (7.00 mM, 3.54 eq., 0.61 mL/min) in a T-piece. The combined mixture was passed through a reactor coil at 60 °C with a flowrate of 2.62 mL/min and a residence time of 60 min. The outlet was collected in a 90 mL CSTR with a stirring rate of 500 rpm affording an average product residence time of 15 min. To the CSTR was continuously added aq. HCl (3.77 mM, 10.56 eq., 3.38 mL/min). The product mixture (suspension) was continuously collected at the CSTR outlet at a flowrate of 6.00 mL/min. The suspension was filtered and the isolated solid was washed successively with Milli-Q water (3 x 5 vol). After crude drying on the filter the desired compound was dried at 40 °C until constant weight. A quantitative yield of product was obtained when executing the run at steady state.

In a combined workflow of Method A1 + Method B2 a total yield of 81% of compound (I-a) was obtained.

While certain features of the invention have been illustrated and described herein, many modifications, substitutions, changes, salts and equivalents will now occur to those of ordinary skill in the art. It is, therefore, to be understood that the appended embodiments are intended to cover all such modifications and changes as fall within the true spirit of the invention.

## Claims

1. A method of producing a solid pharmaceutical composition comprising sodium *N*-(8[2-hydroxybenzoyl]-amino)caprylate (SNAC), comprising the steps of
a) producing a compound of formula (I) 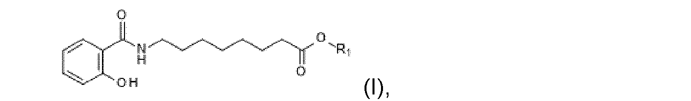
wherein R1 is selected from hydrogen, C1-C6-alkyl, phenyl, benzyl, Mg²⁺, Ca²⁺, Li⁺, Na⁺ and K⁺,
comprising a step of reacting a compound of formula (II), 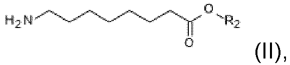
wherein R2 is selected from hydrogen, C1-C6-alkyl, phenyl, benzyl, Mg²⁺, Ca²⁺, Li⁺, Na⁺ and K⁺,
and a compound of formula (III), 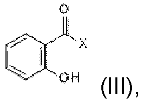
wherein X is selected from O-alkyl, O-phenyl, O-benzyl and N-hydroxysuccinimide ester,
b) producing sodium N-(8[2-hydroxybenzoyl]-amino)caprylate (SNAC) using the compound of formula (I), obtained in a) and
c) preparing said solid pharmaceutical compositions using the sodium N-(8[2-hydroxybenzoyl]-amino)caprylate obtained in step b).

2. The method according to claim 1, wherein SNAC is produced by
b1) mixing the compound of formula (I) obtained in a) with 2-propanol and aqueous sodium hydroxide and
b2) precipitating sodium N-(8[2-hydroxybenzoyl]-amino)caprylate (SNAC) from the mixture of b1).

3. The method according to claim 1 or 2, wherein R₁ is hydrogen or K⁺.

4. The method according to any of the previous claims, wherein R₂ is hydrogen or K⁺.

5. The method according to any of the previous claims 3, wherein X is O-alkyl, such as O-methyl or O-ethyl.

6. The method according to any of the previous claims, wherein X is O-methyl.

7. The method according to any of the previous claims wherein the compound of formular I is 8-[(2-hydroxybenzoyl)amino]octanoic acid of formular (I-a)

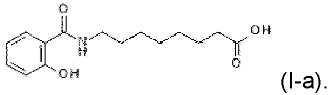

8. The method according to of the previous claims wherein the compound of formular II is 8-amino caprylic acid of formular (II-a)

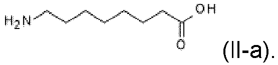

9. The method according to of the previous claims wherein the compound of formular III is methyl salicylate of formular (Illa)

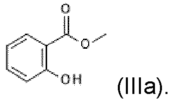

10. The method according to claim 1, wherein the compound of formula I is 8-[(2-hydroxybenzoyl)amino]octanoic acid of formula (I-a), 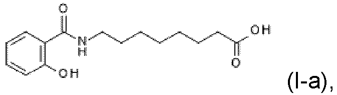
and the method comprises a step of reacting 8-amino caprylic acid of formula (II-a), 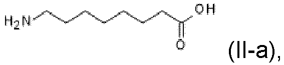
and methyl salicylate of formula (III-a),

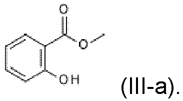

11. The method according to any of the previous claims, wherein potassium 8-[(2-hydroxybenzoyl)amino]octanoate of formula (I-b), 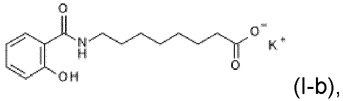 is obtained as an intermediate.

12. The method according to any of the previous claims, wherein the method of producing 8-[(2-hydroxybenzoyl)amino]octanoic acid (I-a) is performed in batch mode.

13. The method according to any of the previous claims 1-11, wherein the method of a) is performed as a continuous process.
